# EUROPÄISCHE PATENTANMELDUNG

(11) **EP 4 335 922 A1**
(43) Veröffentlichungstag der Anmeldung: **13.03.2024**
(21) Anmeldenummer: 23184497.8
(22) Anmeldetag: 10.07.2023
(51) Int. Cl.: C12N 9/18, C11D 3/386

(54) **WASCH- UND REINIGUNGSMITTEL ENTHALTEND TANNASE I**

(30) Priorität: 06.09.2022 DE 102022209245
(71) Anmelder: Henkel AG & Co. KGaA, 40589 Düsseldorf (DE)
(72) Erfinder: Islam, Shohana, 40474 Düsseldorf (DE); Degering, Christian, 40699 Erkrath (DE); Wieland, Susanne, 41541 Zons/Dormagen (DE); Eichstaedt, Renee Charlott, 50733 Köln (DE); Prueser, Inken, 40215 Düsseldorf (DE); Fernandez, Layla, 50858 Köln (DE); Puls, Michael, 40789 Monheim (DE); Gisin, Jonathan, 40789 Monheim (DE); Wiegandt, Jonna, 40599 Düsseldorf (DE)

(57) **Zusammenfassung**

Die Erfindung liegt auf dem Gebiet der Enzymtechnologie. Die Erfindung betrifft Tannasen, die insbesondere im Hinblick auf den Einsatz in Wasch- und Reinigungsmitteln verwendet werden können, alle hinreichend ähnlichen Tannasen mit einer entsprechend ähnlichen Sequenz zu SEQ ID NO:1 und für sie codierende Nukleinsäuren. Die Erfindung betrifft ferner deren Herstellung sowie Verfahren zur Verwendung dieser Tannasen, deren Verwendung als solche sowie diese enthaltende Mittel, insbesondere Wasch- und Reinigungsmittel.

## Beschreibung

Die Erfindung liegt auf dem Gebiet der Enzymtechnologie. Die Erfindung betrifft Tannasen, die insbesondere im Hinblick auf den Einsatz in Wasch- und Reinigungsmitteln verwendet werden können, alle hinreichend ähnlichen Tannasen mit einer entsprechend ähnlichen Sequenz zu SEQ ID NO:1 und für sie codierende Nukleinsäuren. Die Erfindung betrifft ferner deren Herstellung sowie Verfahren zur Verwendung dieser Tannasen, deren Verwendung als solche sowie diese enthaltende Mittel, insbesondere Wasch- und Reinigungsmittel.

Tannasen (EC 3.1.1.20), auch bekannt als Tanninacetylhydrolase oder Tanninacylhydrolase, sind tanninabbauende bzw. tanninhydrolysierende Enzyme, die in vielen Pflanzen, Pilzen oder Bakterien vorkommen. Tannasen zählen zu den Hydrolasen und katalysieren die Hydrolyse von Ester- und Depsidbindungen von hydrolysierbaren Tanninen, insbesondere Gallotanninen, komplexen Tanninen und Gallussäureestern, um Glucose und Gallussäure oder Ellagsäure freizusetzen. Nicht zu den hydrolysierbaren Tanninen zählen die kondensierten Tannine. Unter den Tannasen sind zwei Unterfamilien bekannt: TanA und TanB. Zusätzlich gibt auch einige Feruloylesterasen (EC 3.1.1.73), die Tannase-Aktivität aufweisen können.

Tannine sind sekundäre Pflanzenstoffe, die in vielen Pflanzen wegen ihres bitteren Geschmacks als Schutz vor Fressfeinden enthalten sind. Zu den tanninhaltigen Pflanzen zählen u.a. auch viele Pflanzen, die in der Lebensmittelindustrie Verwendung finden, z.B. Obst, insbesondere Beerenobst wie z.B. Heidelbeeren, Johannisbeeren, Himbeeren und Brombeeren, Fruchtsäfte, Nüssen, Hülsenfrüchte, Tea, Kaffee, Wein, Kakao, Schokolade. Tannine können in 4 unterschiedliche Kategorien eingeteilt werden: Gallotannine (Polygalloylestervon Glucose), Ellagitannine (zusammengesetzt aus Biaryleinheiten und Glucose), komplexe Tannine (eine Catechin-Einheit ist glykosidisch an ein Gallotannin oder eine Ellagitannin-Einheit gebunden) und kondensierte Tannine (Proanthocyanidine, polyflavonoide Tannine, Tannine vom Catechin-Typ, Tannine vom Pyrocatechol-Typ, nicht hydrolysierbare Tannine oder Flavolane).

Wegen ihrer tanninabbauenden Eigenschaften finden Tannasen Anwendung in der Lebensmittelindustrie, um den durch Tannine hervorgerufenen bitteren Geschmack zu reduzieren.

Tanninhaltige Lebensmittel, wie z.B. Obst, insbesondere Beerenobst wie z.B. Heidelbeeren, Johannisbeeren, Himbeeren und Brombeeren, Fruchtsäfte, Nüssen, Hülsenfrüchte, Tea, Kaffee, Wein, Kakao, Schokoladen, können zu hartnäckigen Verschmutzungen auf Textilien und/oder harten Oberflächen, insbesondere Geschirr, führen. Es ist oft schwierig derartige, meist farbige, Flecken/Verschmutzungen effektiv aus verschmutzter Wäsche oder von einem verschmutzten Gegenstand zu entfernen. Farbige Verschmutzungen werden in gängigen Wasch- und Reinigungsmittel üblicherweise mittels Bleichmittel und/oder weiterer reinigungsaktiver Substanzen im Wasch- und Reinigungsmittel, wie z.B. Phosphonate und/oder Phosphate, adressiert. Die genannten Schwierigkeiten bei der Entfernung solcher Flecken und Anschmutzungen sind jedoch besonders gravierend bei der Formulierung flüssiger Wasch- und Reinigungsmittel oder bei der Formulierung von Waschmitteln für farbige Textilien, da diese üblicherweise keine Bleichmittel enthalten. Aufgrund von Nachhaltigkeitsstreben und Bedenken hinsichtlich der Umweltverträglichkeit von Phosphaten und Phosphonaten in Wasch- und Reinigungsmittel werden zunehmend mehr Wasch- und Reinigungsmittel entwickelt, die wenig(er) bis keine Phosphat- und/oder phosphonathaltige Verbindungen enthalten.

Es besteht daher Bedarf Wasch- und Reinigungsmittel, insbesondere flüssige Wasch- und Reinigungsmittel, insbesondere solche ohne Bleichmittel und/oder ohne Phosphonate und/oder Phosphate, dahingehend weiterzuentwickeln, dass sie im Hinblick auf die Entfernung bleichbarer Anschmutzungen, die insbesondere von Früchten, Fruchtsäften, Nüssen, Hülsenfrüchten, Tee, Kaffee, Wein, Kakao oder Schokolade hervorgerufen werden, bevorzugt tanninhaltiger Anschmutzungen, verbessert sind.

Überraschenderweise wurde gefunden, dass eine bislang unbekannte Tannase aus *Streptomyces ipomoeae* oder hierzu hinreichend ähnliche Tannasen (bezogen auf die Sequenzidentität), besonders für den Einsatz in Wasch- oder Reinigungsmitteln, insbesondere flüssigen Wasch- oder Reinigungsmitteln, geeignet ist, da sie ein breites Spektrum an bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, unter Standard-Waschbedingungen entfernt.

Gegenstand der Erfindung ist daher eine Tannase, umfassend eine Aminosäuresequenz, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität mit der in SEQ ID NO:1 angegebenen Aminosäuresequenz über deren Gesamtlänge aufweist, oder Varianten davon.

Die erfindungsgemäßen Varianten sind dadurch gekennzeichnet, dass sie aus einer Tannase, die eine Aminosäuresequenz mit mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität mit der in SEQ ID NO:1 angegebenen Aminosäuresequenz über deren Gesamtlänge aufweist, als Ausgangsmolekül durch ein- oder mehrfache konservative Aminosäuresubstitution erhältlich sind. Alternativ oder ergänzend sind die erfindungsgemäßen Varianten dadurch gekennzeichnet, dass sie aus einer Tannase, die eine Aminosäuresequenz mit mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität mit der in SEQ ID NO:1 angegebenen Aminosäuresequenz über deren Gesamtlänge aufweist, als Ausgangsmolekül durch Fragmentierung, Deletions-, Insertions- oder Substitutionsmutagenese erhältlich sind und eine Aminosäuresequenz umfasst, die über eine Länge von mindestens 200, 210, 220, 230, 240, 250, 260, 270, 280, 290, 300, 310, 320, 330,340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit dem Ausgangsmolekül übereinstimmt.

Ein weiterer Gegenstand der Erfindung ist ein Verfahren zur Herstellung einer Tannase, umfassend das Bereitstellen einer Ausgangstannase, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität zu der in SEQ ID NO:1 angegebenen Aminosäuresequenz über deren Gesamtlänge aufweist oder Varianten der Ausgangstannase, wobei die Varianten wie vorstehend definiert sind.

Eine Tannase im Sinne der vorliegenden Patentanmeldung umfasst daher sowohl die Tannase als solche als auch eine mit einem erfindungsgemäßen Verfahren hergestellte Tannase. Alle Ausführungen zur Tannase beziehen sich daher sowohl auf die Tannase als solche wie auch auf die mittels entsprechender Verfahren hergestellten Tannasen, sowie auf die entsprechenden Verfahren, insbesondere Herstellungsverfahren der Tannase.

Weitere Aspekte der Erfindung betreffen die für diese Tannasen codierenden Nukleinsäuren, erfindungsgemäße Tannasen oder Nukleinsäuren enthaltende nicht-menschliche Wirtszellen sowie erfindungsgemäße Tannasen umfassende Mittel, insbesondere Wasch- und Reinigungsmittel, insbesondere flüssige Wasch- und Reinigungsmittel, Wasch- und Reinigungsverfahren, und Verwendungen der erfindungsgemäßen Tannasen in Wasch- oder Reinigungsmitteln zur Entfernung von bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichtsprozent (Gew.-%).

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens ein(e)", wie hierin verwendet, bedeutet ein(e) oder mehrere, d.h. 1, 2, 3, 4, 5, 6, 7, 8, 9, 10, 11, 12, 13, 14 oder mehr.

Der Begriff "Wasch- und Reinigungsmittel" bzw. "Wasch- oder Reinigungsmittel", wie hierin verwendet, ist gleichbedeutend mit dem Begriff "Mittel" und bezeichnet eine Zusammensetzung zum Reinigen von Textilien und/oder harten Oberflächen, insbesondere Geschirr, wie in der Beschreibung erläutert.

"Etwa", "ca." oder "ungefähr", wie hierin in Bezug auf einen Zahlenwert verwendet, beziehen sich auf den entsprechenden Zahlenwert ±10%, vorzugsweise ±5%.

"Im Wesentlichen frei von" bedeutet, dass die Zusammensetzung bzw. das Mittel weniger als 2 Gew.-%, vorzugsweise weniger als 1 Gew.-%, weiter bevorzugt weniger als 0,5 Gew.-% und besonders bevorzugt weniger als 0,1 Gew.-%, der entsprechenden Substanz, bezogen auf das Gesamtgewicht der Zusammensetzung/des Mittels, enthält.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen. Ein Stoff, z.B. eine Zusammensetzung oder ein Mittel ist gemäß Definition der Erfindung festförmig, wenn sie bei 25°C und 1013 mbar im festen Aggregatzustand vorliegt. Ein Stoff, z.B. eine Zusammensetzung oder ein Mittel ist gemäß Definition der Erfindung flüssig, wenn sie bei 25°C und 1013 mbar im flüssigen Aggregatzustand vorliegt. Dabei umfasst flüssig auch gelförmig.

"Variante", wie hierin verwendet, bezieht sich auf natürliche oder artifiziell erzeugte Variationen einer nativen Tannase, die eine gegenüber der Referenzform abgewandelte Aminosäuresequenz aufweist.

Die vorliegende Erfindung basiert auf der überraschenden Erkenntnis der Erfinder, dass eine erfindungsgemäße Tannase aus *Streptomyces,* insbesondere *Streptomyces ipomoeae,* die eine zu der in SEQ ID NO:1 angegebenen Aminosäuresequenz zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identische Aminosäuresequenz umfasst, die Entfernung von bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, unter Standard-Waschbedingungen bewirkt. Das ist insbesondere insoweit überraschend, als dass bisher keine Tannase, insbesondere keine Tannase aus *Streptomyces,* insbesondere *Streptomyces ipomoeae,* für die Verwendung in Wasch- oder Reinigungsmitteln beschrieben wurde.

In bevorzugten Ausführungsformen ist die erfindungsgemäße Tannase eine Tannase, die tanninabbauende Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch ist. Erfindungsgemäße Tannase-Varianten sind aus einer Tannase mit der angegebenen Sequenzidentität als Ausgangsmolekül durch ein- oder mehrfache konservative Aminosäuresubstitution erhältlich. Alternativ oder ergänzend sind die eingesetzten Varianten aus einer Tannase mit der angegebenen Sequenzidentität als Ausgangsmolekül durch Fragmentierung, Deletions-, Insertions- oder Substitutionsmutagenese erhältlich und umfassen eine Aminosäuresequenz, die über eine Länge von mindestens 200, 210, 220, 230, 240, 250, 260, 270, 280, 290, 300,310, 320, 330, 340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit dem Ausgangsmolekül übereinstimmt.

In bevorzugten Ausführungsformen führt die Verwendung erfindungsgemäßer Tannasen in einem Wasch- oder Reinigungsmittel, vorzugsweise einem flüssigen Wasch- oder Reinigungsmittel, zu einer verbesserten Reinigungsleistung dieser Mittel, an mindestens einer und zunehmend bevorzugt zwei, drei vier, fünf, sechs oder sieben tannasesensitiven Anschmutzung(en), die vorzugsweise aus der aus bleichbaren und/oder tanninhaltiger Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind. Erfindungsgemäße Tannasen ermöglichen folglich eine verbesserte Entfernung von mindestens einer, bevorzugt von mehreren, tannasesensitiven Anschmutzung(en) auf Textilien und/oder harten Oberflächen, beispielsweise Geschirr. Typische tannasesensitive Anschmutzungen umfassen beispielsweise bleichbare und/oder tanninhaltige Anschmutzungen, die insbesondere von Früchten, Fruchtsäften, Nüssen, Hülsenfrüchten, Tee, Kaffee, Wein, Kakao oder Schokolade hervorgerufen werden. Eine erfindungsgemäße Verbesserung der Reinigungsleistung, insbesondere der tanninabbauenden Reinigungsleistung, liegt dann vor, wenn die Tannase an mindestens einer und zunehmend bevorzugt an zwei, drei, vier, fünf, sechs oder sieben tannasesensitiven Anschmutzung(en), die vorzugsweise aus der aus bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind, eine verbesserte Reinigungsleistung gegenüber einem Mittel ohne Tannase zeigt, wie hierin beschrieben.

In bevorzugten Ausführungsformen ist die erfindungsgemäße Tannase eine Tannase, die tanninabbauende Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch ist, wobei die Tannase an mindestens einer und zunehmend bevorzugt an zwei, drei, vier, fünf, sechs oder sieben tannasesensitiven Anschmutzung(en), die vorzugsweise aus der aus bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind, eine verbesserte Reinigungsleistung eines Wasch- oder Reinigungsmittels, welches eine erfindungsgemäße Tannase enthält, gegenüber einem Mittel ohne Tannase bewirkt, wobei die Reinigungsleistung, wie in Beispiel 2 beschrieben, bestimmt wird.

Solche leistungsverbesserten Waschergebnisse an tannasesensitiven Anschmutzungen können in verschiedenen Temperaturbereichen erzielt werden, z.B. in einem Bereich von etwa 0°C bis etwa 100°C, bevorzugt etwa 20°C bis etwa 60°C, insbesondere etwa 20°C bis etwa 40°C.

Die erfindungsgemäßen Tannasen verfügen über eine hohe Stabilität in Wasch- oder Reinigungsmitteln, beispielsweise gegenüber Tensiden und/oder Bleichmitteln und/oder Chelatoren und/oder gegenüber Temperatureinflüssen, insbesondere gegenüber hohen Temperaturen, beispielsweise zwischen etwa 50°C und etwa 65°C, insbesondere etwa 60°C, und/oder gegenüber sauren oder alkalischen Bedingungen und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. Mit besonders bevorzugten Ausführungsformen der Erfindung werden folglich leistungsverbesserte Tannase-Varianten bereitgestellt. Solche vorteilhaften Ausführungsformen erfindungsgemäßer Tannasen ermöglichen folglich verbesserte Waschergebnisse an bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, in einem weiten Temperaturbereich.

Unter Reinigungsleistung wird im Rahmen der Erfindung die Aufhellungsleistung an einer oder mehreren Anschmutzungen, insbesondere auf Wäsche oder Geschirr, verstanden. Im Rahmen der Erfindung weist sowohl das Wasch- oder Reinigungsmittel, welches die Tannase umfasst bzw. die durch dieses Mittel gebildete Wasch- oder Reinigungsflotte, als auch die Tannase selbst eine jeweilige Reinigungsleistung auf. Die Reinigungsleistung des Enzyms trägt somit zur Reinigungsleistung des Mittels bzw. der durch das Mittel gebildeten Wasch- oder Reinigungsflotte bei. Die Reinigungsleistung wird bevorzugt ermittelt wie weiter unten angegeben.

Unter Waschflotte wird diejenige das Wasch- oder Reinigungsmittel enthaltende Gebrauchslösung verstanden, die auf Textilien oder Gewebe bzw. harte Oberflächen einwirkt und damit mit den auf Textilien bzw. Geweben oder harten Oberflächen vorhandenen Anschmutzungen in Kontakt kommt. Üblicherweise entsteht die Waschflotte, wenn der Wasch- oder Reinigungsvorgang beginnt und das Wasch- oder Reinigungsmittel beispielsweise in einer Geschirrspülmaschine, einer Waschmaschine oder in einem anderen geeigneten Behältnis mit Wasser verdünnt wird.

Die erfindungsgemäße Tannase weist eine enzymatische Aktivität auf, d.h. sie ist zur Hydrolyse von Tanninen befähigt, insbesondere in einem Wasch- und Reinigungsmittel. Vorteilhafterweise weist das erfindungsgemäße Wasch- und Reinigungsmittel eine verbesserte Reinigungsleistung, insbesondere bei der Entfernung von bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, auf. Besonders bevorzugt eignet sich das erfindungsgemäße Wasch- und Reinigungsmittel zur Entfernung von bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden.

Ferner handelt es sich bei einer erfindungsgemäßen Tannase vorzugsweise um eine reife (mature) Tannase, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Tannase ein frei vorliegendes Enzym. Dies bedeutet, dass die Tannase mit allen Komponenten eines Mittels direkt agieren kann und, falls es sich bei dem Mittel um ein Flüssigmittel handelt, dass die Tannase direkt mit dem Lösungsmittel des Mittels (z.B. Wasser) in Kontakt steht. In anderen Ausführungsformen kann ein Mittel Tannasen enthalten, die einen Interaktionskomplex mit anderen Molekülen bilden oder die eine "Umhüllung" enthalten. Hierbei kann ein einzelnes oder mehrere Tannase-Molekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von z.B. *Bacillus pumilus* oder *Bacillus subtilis* oder anderen Expressionsstämmen, die die erfindungsgemäße Tannase exprimieren, oder Zellkulturüberstände solcher Zellen enthalten.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) Basic local alignment search tool, J. Mol. Biol., 215:403-410, und Altschul et al. (1997) Gapped BLAST and PSI-BLAST: a new generation of protein database search programs, Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) Multiple sequence alignment with the Clustal series of programs, Nucleic Acid Res., 31 :3497-3500), T-Coffee (vgl. z.B. Notredame et al. (2000) T-Coffee: A novel method for multiple sequence alignments, J. Mol. Biol., 302:205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert, oder Clone Manager 10 (Verwendung der Scoring Matrix BLOSUM 62 für Sequenz-Alignment auf Aminosäureebene). Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist. Weiterhin richtet sich die Zuordnung der Positionen nach dem reifen (maturen) Protein. Diese Zuordnung ist insbesondere auch anzuwenden, wenn die Aminosäuresequenz einer Tannase eine höhere Zahl von Aminosäureresten umfasst als die Tannase gemäß SEQ ID NO:1.

In einer weiteren Ausführungsform können erfindungsgemäße Tannasen Aminosäureveränderungen, insbesondere Aminosäure-Substitutionen, -Insertionen oder -Deletionen, aufweisen. Solche Tannasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Enzyme oder anderer Polypeptide genutzt werden. Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Tannase erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Tannase, z.B. hinsichtlich ihrer Aktivität und/oder ihrer Toleranz in Bezug auf das Substratspektrum, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere oder alternative Austausche innerhalb derselben Polypeptidkette werden durch Schrägstriche voneinander getrennt. "130D/V" bedeutet somit, dass die Position 130 zu D oder V mutiert ist. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Ein weiterer Gegenstand der Erfindung ist daher eine Tannase, die dadurch gekennzeichnet ist, dass sie aus einer Tannase wie hierin beschrieben als Ausgangsmolekül erhältlich ist durch ein- oder mehrfache konservative Aminosäuresubstitution. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T. Dabei kann die Tannase vor und/oder nach der konservativen Aminosäuresubstitution eine Aminosäuresequenz umfassen, die zu der in SEQ ID NO:1 angegebenen Aminosäuresequenz über deren Gesamtlänge zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch ist.

Alternativ oder ergänzend ist die Tannase dadurch gekennzeichnet, dass sie aus einer erfindungsgemäßen Tannase als Ausgangsmolekül erhältlich ist durch Fragmentierung, Deletions-, Insertions- oder Substitutionsmutagenese und eine Aminosäuresequenz umfasst, die über eine Länge von mindestens 200, 210, 220, 230, 240, 250, 260, 270, 280, 290, 300,310, 320, 330,340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit dem Ausgangsmolekül übereinstimmt. Dabei kann die Tannase vor und/oder nach der Fragmentierung, Deletions-, Insertions- oder Substitutionsmutagenese eine Aminosäuresequenz umfassen, die zu der in SEQ ID NO:1 angegebenen Aminosäuresequenz über deren Gesamtlänge zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch ist.

So ist es beispielsweise möglich, an den Termini oder in den Loops des Enzyms einzelne Aminosäuren zu deletieren, ohne dass dadurch die katalytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre katalytische Aktivität, d.h. ihre katalytische Aktivität entspricht mindestens derjenigen des Ausgangsenzyms, d.h. in einer bevorzugten Ausführungsform beträgt die katalytische Aktivität mindestens 80%, vorzugsweise mindestens 90%, weiter bevorzugt mindestens 100% der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Enzymen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Tannase gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Tannase-Variante beobachtet, deren Aminosäureaustausch durch dieselbe eingeführte Aminosäure erreicht wurde, so ist hierin eine Bestätigung der korrekten Zuordnung zu sehen.

Insbesondere werden erfindungsgemäß auch Fragmente der Tannase wie hierin definiert, insbesondere solche gemäß SEQ ID NO:1, die am N- und/oder C-Terminus derart verkürzt sind, dass ein oder mehrere Aminosäuren der Tannase, beispielsweise 1, 2, 3, 4, 5, 6, 7, 8, 9 oder 10, nicht länger enthalten sind, erfasst. Auch von diesen verkürzten Fragmenten können in verschiedenen Ausführungsformen der Erfindung Varianten verwendet werden, die zu der ausgehend von der in SEQ ID NO:1 angegebenen Aminosäuresequenz um (jeweils) 1 bis 10 N-terminale und/oder C-terminale Aminosäuren verkürzten Form, über die Gesamtlänge zu mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch sind. Beispielsweise werden erfindungsgemäß auch Tannasen erfasst, die eine Aminosäuresequenz aufweisen, die über die Tannase umfassend eine Aminosäuresequenz, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität mit der in SEQ ID NO:1 angegebenen Aminosäuresequenz über deren Gesamtlänge aufweist bzw. den hierin beschriebenen Varianten davon, hinausgeht, ohne dass dadurch die katalytische Aktivität verloren oder vermindert wird. Vorzugsweise sind derartige Tannasen solche, die N- und/oder C-terminal zusätzliche Aminosäuren, beispielsweise das Signal-Peptid oder Fragmente des Signal-Peptids aufweisen, wobei das Signal-Peptid oder die Fragmente des Signal-Peptids bei der Herstellung der Tannase entstehen.

Alle genannten Sachverhalte sind auch auf die erfindungsgemäßen Verfahren zur Herstellung einer Tannase anwendbar.

Demnach umfasst ein erfindungsgemäßes Verfahren ferner einen oder mehrere der folgenden Verfahrensschritte:
(a) Bereitstellen einer Ausgangstannase, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität zu der in SEQ ID NO:1 angegebenen Aminosäuresequenz über deren Gesamtlänge aufweist, oder Varianten davon;
(b) Einbringen einer ein- oder mehrfachen konservativen Aminosäuresubstitution;
(c) Veränderung der Aminosäuresequenz durch Fragmentierung, Deletions-, Insertions- oder Substitutionsmutagenese derart, dass die Tannase eine Aminosäuresequenz umfasst, die über eine Länge von mindestens 200, 210, 220, 230, 240, 250, 260, 270, 280, 290, 300,310, 320, 330,340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit dem Ausgangsmolekül übereinstimmt.

Sämtliche Ausführungsformen gelten auch für die erfindungsgemäßen Verfahren.

In einer weiteren Ausführungsform der Erfindung ist eine zuvor beschriebene Tannase stabilisiert, insbesondere durch eine oder mehrere Mutationen, beispielsweise Substitutionen, oder durch Kopplung an ein Polymer. Eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

In einer weiteren Ausführungsform der Erfindung ist die Tannase dadurch gekennzeichnet, dass ihre Reinigungsleistung gegenüber dem Wildtypenzym (SEQ ID NO:1) nicht signifikant verringert ist, d.h. mindestens 70% und zunehmend bevorzugt 75%, 80%, 85%, 90% oder 95% der Referenzwaschleistung besitzt, und weiter bevorzugt mindestens 100%, noch bevorzugter mindestens 110%, besonders bevorzugt mindestens 120% oder mehr.

Die Reinigungsleistung kann in einem Waschsystem bestimmt werden, das ein Waschmittel in einer Dosierung zwischen 2,0 und 8,0 Gramm pro Liter Waschflotte sowie das Enzym enthält. Die zu vergleichenden Enzyme werden konzentrationsgleich (bezogen auf aktives Protein) eingesetzt. Durch den aktivitätsgleichen Einsatz der jeweiligen Enzyme wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Die Konzentration der Tannase in dem für ein solches Waschsystem bestimmten Waschmittel beträgt 0,001 bis 0,1 Gew.-%, vorzugsweise 0,01 bis 0,06 Gew.-%, bezogen auf aktives Protein.

Unter Wasch- bzw. Reinigungsleistung wird das Vermögen eines Wasch- oder Reinigungsmittels, eine vorhandene Anschmutzung teilweise oder vollständig zu entfernen, insbesondere die Aufhellungsleistung an einer oder mehreren Anschmutzungen auf Textilien, insbesondere Baumwolltextilien, Polyestertextilien und/oder Baumwoll-Polyester-Mischtextilien, verstanden. Beispiele für solche Anschmutzungen sind Blut auf Baumwolle oder Schokolade-Milch/Ruß auf Baumwolle, Kakao auf Baumwolle, Eigelb auf Baumwolle, Milch/Ruß auf Baumwolle oder Porridge auf Baumwolle usw. Weitere Anschmutzungen umfassen bleichbare und/oder tanninhaltige bzw. tanninderivathaltige Anschmutzungen, die insbesondere von Früchten, Fruchtsäften, Nüssen, Hülsenfrüchten, Tee, Kaffee, Wein, Kakao oder Schokolade hervorgerufen werden. Weitere Beispiele umfassen die vorgenannten Anschmutzungen auch auf Baumwoll-Polyester-Mischtextilien oder polyesterhaltigen Textilien oder anderen Mischtextilien. Im Rahmen der Erfindung weisen sowohl das Wasch- oder Reinigungsmittel, welches die Tannase umfasst, bzw. die durch dieses Mittel gebildete Wasch- bzw. Reinigungsflotte, als auch die Tannase selbst eine jeweilige Reinigungsleistung auf. Die Reinigungsleistung der Tannase trägt somit zur Reinigungsleistung des Mittels bzw. der durch das Mittel gebildeten Wasch- bzw. Reinigungsflotte bei.

Unter Wasch- bzw. Reinigungsflotte wird diejenige das Wasch- oder Reinigungsmittel enthaltende Gebrauchslösung verstanden, die auf die Textilien bzw. harten Oberflächen einwirkt und damit mit den auf den Textilien bzw. harten Oberflächen vorhandenen Anschmutzungen in Kontakt kommt. Üblicherweise entsteht die Wasch- bzw. Reinigungsflotte, wenn der Wasch- bzw. Reinigungsvorgang beginnt und das Wasch- oder Reinigungsmittel z.B. in einer Wasch- oder Spülmaschine oder in einem anderen geeigneten Behältnis mit Wasser verdünnt wird.

Ein flüssiges Referenzwaschmittel für ein solches Waschsystem kann z.B. wie folgt zusammengesetzt sein (alle Angaben in Gewichts-Prozent (Gew.-%)): 4,4% Alkylbenzolsulfonsäure, 5,6% weitere anionische Tenside, 2,4% C₁₂₋₁₈ Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 7 und pH 10,5, bevorzugt zwischen pH 8 und pH 9.

Die Reinigungsleistung wird gegenüber einer Anschmutzung auf Baumwolle durch Messung des Reinigungsgrades der gewaschenen Textilien bestimmt. Beispielsweise kann der Waschvorgang für 60 Minuten bei einer Temperatur von etwa 20°C oder etwa 40°C erfolgen und das Wasser eine Wasserhärte zwischen 15,5°dH und 16,5°dH (deutsche Härte) aufweisen. Im Rahmen der Erfindung erfolgt die Bestimmung der Reinigungsleistung beispielsweise bei 20°C oder 40°C unter Verwendung eines flüssigen Waschmittels wie z.B. des vorstehend angegebenen, wobei der Waschvorgang vorzugsweise für 60 Minuten bei 600 rpm erfolgt.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Bevorzugte Ausführungsformen erfindungsgemäßer Tannasen erzielen solche vorteilhaften Reinigungsleistungen auch schon bei niedrigen Temperaturen, insbesondere in den Temperaturbereichen zwischen etwa 10°C und etwa 60°C, bevorzugt zwischen etwa 15°C und etwa 50°C und besonders bevorzugt zwischen etwa 20°C und etwa 40°C.

Verfahren zur Bestimmung der Tannase-Aktivität sind dem Fachmann auf dem Gebiet der Enzymtechnologie geläufig und werden von ihm routinemäßig angewendet, z.B. Tanninsaure-Assay nach Mondal et al. (Colorimetric Assay Method for Determination of the Tannin Acyl Hydrolase (EC 3.1.1.20) Activity; Analytical Biochemistry 295, 168-171, 2001) oder Rhodanin-Assay nach Sharma et al. (A Spectrophotometric Method for Assay of Tannase using Rhodanine; Analytical Biochemistry 279, 85-89, 2000).

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (Gornall et al., J. Biol. Chem. 177 (1948): 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al., J. Am. Chem. Soc. 88, 24 (1966): 5890-5913) erfolgen.

Ein weiterer Gegenstand der Erfindung ist eine Tannase wie hierin beschrieben, die dadurch gekennzeichnet ist, dass sie mindestens eine chemische Modifikation aufweist. Eine Tannase mit einer solchen Veränderung wird als Derivat bezeichnet, d.h. die Tannase ist derivatisiert. Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Tannasen mit spezifischen Inhibitoren ist diesbezüglich möglich. Unter allen hierin beschriebenen Tannasen beziehungsweise Tannase-Varianten und/oder-Derivaten sind im Rahmen der vorliegenden Erfindung diejenigen besonders bevorzugt, deren Lagerstabilität und/oder katalytische Aktivität und/oder Substrattoleranz und/oder Reinigungsleistung derjenigen Tannase gemäß SEQ ID NO:1 entspricht und/oder gegenüber der Tannase gemäß SEQ ID NO:1 verbessert ist, wobei die katalytische Aktivität und/oder Reinigungsleistung wie hierin beschrieben bestimmt wird.

Die erfindungsgemäßen und die in den erfindungsgemäßen Waschmitteln einsetzbaren Tannasen sind aus Pflanzen, Pilzen und/oder Bakterien erhältlich. Die die SEQ ID NO:1 umfassende Tannase kann aus *Streptomyces ipomoeae* gewonnen werden.

Die natürlichen Produktionsmengen der Tannasen sind allerdings oft sehr niedrig. Es kann daher sinnvoll sein, die Produktion zu erhöhen, indem man Tannase-Gene in fremden Produktionswirten exprimiert. Zu diesem Zweck verwendet man in der Regel Vektoren, die eine Nukleinsäure enthalten, die für eine erfindungsgemäße Tannase codiert.

Ein weiterer Gegenstand der Erfindung ist eine Nukleinsäure, die für eine erfindungsgemäße Tannase codiert, sowie ein Vektor enthaltend eine solche Nukleinsäure, insbesondere ein Klonierungsvektor oder ein Expressionsvektor. Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesem Erfindungsgegenstand eingeschlossen, die eine der vorstehend beschriebenen Tannasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden. Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren. Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht-menschliche Wirtszelle, die eine erfindungsgemäße Nukleinsäure oder einen erfindungsgemäßen Vektor beinhaltet, oder die eine erfindungsgemäße Tannase beinhaltet, insbesondere eine, die die Tannase in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Tannasen von den sie produzierenden Zellen nach deren Herstellung modifiziert werden, beispielsweise durch Anknüpfung von Zuckermolekülen, Formylierungen, Aminierungen, usw. Solche posttranslationalen Modifikationen können die Tannase funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein. Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist. Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren. Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar. In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Co-Expression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Tannasen herzustellen. Ein weiterer Gegenstand der Erfindung ist daher ein Verfahren zur Herstellung einer Tannase umfassend
a) Kultivieren einer erfindungsgemäßen Wirtszelle, und
b) Isolieren der Tannase aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Tannasen. Alle Fermentationsverfahren, die auf einem entsprechenden Verfahren zur Herstellung einer erfindungsgemäßen Tannase beruhen, stellen Ausführungsformen dieses Erfindungsgegenstandes dar. Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Tannase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden. Die hergestellte Tannase kann aus dem Fermentationsmedium geerntet werden. Ein solches Fermentationsverfahren ist gegenüber einer Isolation der Tannase aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Tannase in das Fermentationsmedium sezernieren. Ohne Sekretion kann alternativ die Isolation der Tannase aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Tannase herzustellen.

Ein weiterer Gegenstand der Erfindung ist ein Mittel, das dadurch gekennzeichnet ist, dass es eine erfindungsgemäße Tannase wie hierin beschrieben enthält. Bevorzugt ist das Mittel ein Wasch- oder Reinigungsmittel, weiter bevorzugt ein flüssiges Wasch- oder Reinigungsmittel, besonders bevorzugt flüssiges Waschmittel.

Besonders bevorzugt ist das Wasch- und Reinigungsmittel im Wesentlichen frei von borhaltigen Verbindungen. "Im Wesentlichen frei von borhaltigen Verbindungen" bedeutet in diesem Zusammenhang, dass die erfindungsgemäßen Mittel weniger als 2 Gew.-%, vorzugsweise weniger als 1 Gew.-%, weiter bevorzugt weniger als 0,5 Gew.-% und besonders bevorzugt weniger als 0,1 Gew.- %, borhaltige Verbindungen, bezogen auf das Gesamtgewicht des Mittels, enthalten. In ganz besonders bevorzugten Ausführungsformen sind die erfindungsgemäßen Wasch- und Reinigungsmittel frei von borhaltigen Verbindungen, d.h. sie enthalten insbesondere keine Borsäure und/oder Phenylboronsäurederivate.

In bevorzugten Ausführungsformen wird die erfindungsgemäße Tannase in Mitteln oder Zusammensetzungen eingesetzt, die im Wesentlichen frei von phosphonathaltigen Verbindungen sind. "Im Wesentlichen frei von phosphonathaltigen Verbindungen" bedeutet in diesem Zusammenhang, dass die entsprechende Mittel oder Zusammensetzungen weniger als 2 Gew.-%, vorzugsweise weniger als 1 Gew.-%, weiter bevorzugt weniger als 0,5 Gew.-% und besonders bevorzugt weniger als 0,1 Gew.-%, phosphonathaltige Verbindungen, bezogen auf das Gesamtgewicht des Mittels/der Zusammensetzung, enthalten. In besonders bevorzugten Ausführungsformen sind diese Mittel/Zusammensetzungen frei von phosphonathaltigen Verbindungen.

In bevorzugten Ausführungsformen wird die erfindungsgemäße Tannase in Mitteln oder Zusammensetzungen eingesetzt, die im Wesentlichen frei von phosphathaltigen Verbindungen sind. "Im Wesentlichen frei von phosphathaltigen Verbindungen" bedeutet in diesem Zusammenhang, dass die entsprechende Mittel oder Zusammensetzungen weniger als 2 Gew.-%, vorzugsweise weniger als 1 Gew.-%, weiter bevorzugt weniger als 0,5 Gew.-% und besonders bevorzugt weniger als 0,1 Gew.- %, phosphathaltige Verbindungen, bezogen auf das Gesamtgewicht des Mittels/der Zusammensetzung, enthalten. In besonders bevorzugten Ausführungsformen sind diese Mittel/Zusammensetzungen frei von phosphathaltigen Verbindungen.

Unter einem Wasch- oder Reinigungsmittel sind erfindungsgemäß alle denkbaren Wasch- oder Reinigungsmittelarten zu verstehen, sowohl Konzentrate als auch unverdünnt anzuwendende Mittel, zum Einsatz im kommerziellen Maßstab, in der Waschmaschine oder bei der Handwäsche bzw. -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen (maschinelle Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige oder granulare Feststoffe, in verdichteter oder nachverdichteter Teilchenform, als homogene Lösungen oder Suspensionen vorliegen können, können neben einer erfindungsgemäßen Tannase alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Polymere, Glaskorrosionsinhibitoren, Korrosionsinhibitoren, Bleichmittel wie Persauerstoffverbindungen, Bleichaktivatoren oder Bleichkatalysatoren enthalten. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten. Vorteilhafte Inhaltsstoffe erfindungsgemäßer Mittel sind offenbart in der internationalen Patentanmeldung WO 2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die erfindungsgemäße Tannase vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. In verschiedenen Ausführungsformen beträgt die Konzentration der hierin beschriebenen Tannase (aktives Enzym) in dem Mittel >0 bis 1 Gew.-%, vorzugsweise 0,0001 oder 0,001 bis 0,1 Gew.-% bezogen auf das Gesamtgewicht des Mittels oder der Zusammensetzung.

Ein erfindungsgemäßes Mittel enthält die Tannase zunehmend bevorzugt in einer Menge von 1 × 10⁻⁸ bis 5 Gew.-%, von 0,0001 bis 1 Gew.-%, von 0,0005 bis 0,5 Gew.-%, von 0,001 bis 0,1 Gew.-%, jeweils bezogen auf aktives Protein und bezogen auf das Gesamtgewicht des Waschmittels.

Die Ausführungsformen der vorliegenden Erfindung umfassen alle festen, pulverförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Flüssige Mittel sind generell bevorzugt. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Die erfindungsgemäßen Tannasen werden bevorzugt in flüssigen Waschmitteln zum Reinigen von Textilien eingesetzt, besonders bevorzugt in flüssigen Waschmitteln mit einem pH-Wert von etwa 8 bis etwa 9.

Erfindungsgemäße Wasch- oder Reinigungsmittel können ausschließlich eine erfindungsgemäße Tannase enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Protease, Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase oder Oxidoreduktase, sowie deren Gemische. Weitere Enzyme sind in dem Mittel vorteilhafterweise jeweils in einer Menge von 1 × 10⁻⁸ bis 5 Gew.-% bezogen auf aktives Protein enthalten. Zunehmend bevorzugt ist jedes weitere Enzym in einer Menge von 1 × 10⁻⁷ bis 3 Gew.-%, von 0,00001 bis 1 Gew.- %, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in dem Mittel enthaltenen Enzyme unterstützen sich in ihrer Reinigungsleistung gegenseitig. Ganz besonders bevorzugt liegt ein solcher Synergismus vor zwischen der erfindungsgemäß enthaltenen Tannase und einem weiteren Enzym eines erfindungsgemäßen Mittels. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Erfindungsgemäß bevorzugte Textilwaschmittel weisen mindestens eine Tannase und mindestens eine Protease auf. In einer weiteren bevorzugten Ausführungsform der Erfindung weisen Textilwaschmittel mindestens eine Tannase und mindestens eine Amylase auf. In einer weiteren bevorzugten Ausführungsform der Erfindung weisen Textilwaschmittel mindestens eine Tannase und mindestens eine Cellulase auf. In einer weiteren bevorzugten Ausführungsform weisen Textilwaschmittel mindestens eine Tannase und mindestens eine Lipase auf. In einer weiteren bevorzugten Ausführungsform weisen Textilwaschmittel mindestens eine Tannase, mindestens eine Protease, mindestens eine Amylase und mindestens eine Lipase auf. In einer weiteren bevorzugten Ausführungsform weisen Textilwaschmittel mindestens eine Tannase, mindestens eine Protease, mindestens eine Amylase und mindestens eine Cellulase auf. In einer weiteren bevorzugten Ausführungsform weisen Textilwaschmittel mindestens eine Tannase, mindestens eine Protease, mindestens eine Amylase, mindestens eine Cellulase und mindestens eine Lipase auf. Besonders bevorzugt sind Textilwaschmittel, welche 3 bis 10 verschiedene Enzyme aufweisen, wobei Textilwaschmittel, welche 3 bis 10 unterschiedliche Enzymarten aufweisen, im Hinblick auf die Reinigungsleistung gegenüber einem sehr großen Fleckenspektrum von besonderer Bevorzugung sein können.

Beispiele für Proteasen sind die Subtilisine BPN' aus *Bacillus amyloliquefaciens* und Carlsberg aus *Bacillus licheniformis,* die Protease PB92, die Subtilisine 147 und 309, die Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von dem Unternehmen Novozymes erhältlich. Die Subtilisine 147 und 309 werden unter den Handelsnamen Esperase^{®} bzw. Savinase^{®} von dem Unternehmen Novozymes vertrieben. Von der Protease aus *Bacillus lentus* DSM 5483 leiten sich Protease-Varianten ab, beschrieben in z.B. WO 95/23221, WO 92/21760 WO 2013/060621 und EP 3660151. Weitere brauchbare Proteasen sind z.B. die unter den Handelsnamen Durazym^{®}, Relase^{®}, Everlase^{®}, Nafizym^{®}, Natalase^{®}, Kannase^{®}, Progress Uno 101L^{®} und Ovozyme^{®} von dem Unternehmen Novozymes, die unter den Handelsnamen, Purafect^{®}, Purafect^{®} OxP, Purafect^{®} Prime, Excellase^{®}, Properase^{®}, Preferenz P100^{®} und Preferenz P300^{®} von dem Unternehmen Danisco/DuPont, das unter dem Handelsnamen Lavergy pro 104 LS^{®} von dem Unternehmen BASF, das unter dem Handelsnamen Protosol^{®} von dem Unternehmen Advanced Biochemicals Ltd., das unter dem Handelsnamen Wuxi^{®} von dem Unternehmen Wuxi Snyder Bioproducts Ltd., die unter den Handelsnamen Proleather^{®} und Protease P^{®} von dem Unternehmen Amano Pharmaceuticals Ltd., und das unter der Bezeichnung Proteinase K-16 von dem Unternehmen Kao Corp. erhältlichen Enzyme. Besonders bevorzugt eingesetzt werden auch die Proteasen aus *Bacillus gibsonii* und *Bacillus pumilus,* die offenbart sind in WO 2008/086916, WO 2007/131656, WO 2017/215925, WO 2021/175696 und WO 2021/175697. Weitere verwendbare Proteasen sind diejenigen, die in den Mikroorganismen *Stenotrophomonas maltophilia,* insbesondere *Stenotrophomonas maltophilia* K279a, *Bacillus intermedius* sowie *Bacillus sphaericus* natürlicherweise vorhanden sind.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl^{®} und von dem Unternehmen Danisco/DuPont unter dem Namen Purastar^{®} ST erhältlich. Weiterentwicklungsprodukte dieser α-Amylase sind unter den Handelsnamen Duramyl^{®} und Termamyl^{®} ultra (beide von Novozymes), Purastar^{®} OxAm (Danisco/DuPont) und Keistase^{®} (Daiwa Seiko Inc.) erhältlich. Die α-Amylase von *Bacillus amyloliquefaciens* wird von dem Unternehmen Novozymes unter dem Namen BAN^{®} vertrieben, und abgeleitete Varianten von der α-Amylase aus *Bacillus stearothermophilus* unter den Namen BSG^{®} und Novamyl^{®}, ebenfalls von dem Unternehmen Novozymes. Des Weiteren sind für diesen Zweck die α-Amylase aus *Bacillus sp.* A 7-7 (DSM 12368) und die Cyclodextrin-Glucanotransferase (CGTase) aus *Bacillus agaradherens* (DSM 9948) hervorzuheben. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus *Aspergillus niger* und A. *oryzae* geeignet. Weitere vorteilhaft einsetzbare Handelsprodukte sind z.B. die Amylase-LT^{®} und Stainzyme^{®} oder Stainzyme^{®} ultra bzw. Stainzyme^{®} plus sowie Amplity^{™} 12L oder Amplify Prime^{™} 100L, letztere ebenfalls von dem Unternehmen Novozymes, sowie die PREFERENZ S^{®} Serie von dem Unternehmen Danisco/DuPont, umfassend z.B. PREFERENZ S100^{®}, PREFERENZ S1000^{®} oder PREFERENZ S210^{®}. Auch durch Punktmutationen erhältliche Varianten dieser Enzyme können erfindungsgemäß eingesetzt werden.

Geeignete Cellulasen umfassen solche bakterieller oder pilzlicher Herkunft. Chemisch modifizierte oder proteintechnisch veränderte Mutanten sind eingeschlossen. Geeignete Cellulasen sind Cellulasen aus den Gattungen *Bacillus, Pseudomonas, Humicola, Fusarium, Thielavia, Acremonium,* z. B. die Pilzcellulasen aus *Humicola insolens, Myceliophthora thermophila* und *Fusarium oxysporum,* die in US 4435307, US 5648263, US 5691178, US 5776757 und WO 89/09259 offenbart sind. Besonders geeignete Cellulasen sind die alkalischen oder neutralen Cellulasen mit farbpflegenden Eigenschaften. Beispiele für solche Cellulasen sind Cellulasen, die in EP 0495257, EP 0531372, WO 96/11262, WO 96/29397 und WO 98/08940 beschrieben sind. Beispiele für Cellulasen mit Endo-1,4-Glucanase-Aktivität (EC 3.2.1.4) sind in WO 2002/099091 beschrieben, z.B. solche mit einer Sequenz von mindestens 97% Identität zu der Aminosäuresequenz der Positionen 1 bis 773 von SEQ ID NO:2 der WO 2002/099091. Ein weiteres Beispiel kann eine GH44-Xyloglucanase umfassen, z.B. ein Xyloglucanase-Enzym mit einer Sequenz von mindestens 60% Identität zu den Positionen 40 bis 559 der SEQ ID NO:2 der WO 2001/062903. Zu den kommerziell verfügbaren Cellulasen gehören Celluzyme^{™}, Carezyme^{™}, Carezyme Premium^{™}, Celluclean^{™} (z.B. Celluclean^{™} 5000L ans Celluclean^{™} 4000T), Celluclean Classic^{™}, Cellusoft^{™}, Endolase^{®}, Renozyme^{®} und Whitezyme^{™} (Novozymes A/S), Clazinase^{™} und Puradax HA^{™} (Genencor International Inc.), KAC-500(B)^{™} (Kao Corporation), Revitalenz^{™} 1000, Revitalenz^{™} 2000 und Revitalenz^{™} 3000 (DuPont), sowie Ecostone^{®} und Biotouch^{®} (AB Enzymes).

Als weitere Enzyme einsetzbar sind z.B. Lipasen oder Cutinasen, insbesondere wegen ihrer Triglycerid-spaltenden Aktivitäten, aber auch, um aus geeigneten Vorstufen *in situ* Persäuren zu erzeugen. Geeignete Lipasen und Cutinasen sind solche bakteriellen oder pilzlichen Ursprungs. Chemisch modifizierte oder durch Proteinengineering erzeugte mutierte Enzyme sind eingeschlossen. Beispiele sind Lipase aus *Thermomyces,* z.B. aus *T. lanuginosus* (früher *Humicola lanuginosa* genannt), wie in EP 0258068 und EP 0305216 beschrieben, Cutinase aus *Humicola,* z.B. *H. insolens* (WO 96/13580), Lipase aus Stämmen von *Pseudomonas* (einige davon jetzt umbenannt in *Burkholderia),* z.B. *P. alcaligenes* oder *P*. *pseudoalcaligenes, P. cepacia, P. sp.* Stamm SD705, *P*. *wisconsinensis, Streptomyces-Lipasen* vom GDSL-Typ, Cutinase aus *Magnaporthe grisea,* Cutinase aus *Pseudomonas mendocina,* Lipase aus *Thermobifida fusca,* Lipase aus *Geobacillus stearothermophilus,* Lipase aus *Bacillus subtilis* und Lipase aus *Streptomyces griseus* und S. *pristinaespiralis.* Zu bevorzugten Lipasen gehören z.B. die ursprünglich aus *Humicola lanuginosa* (*Thermomyces lanuginosus*) erhältlichen bzw. daraus weiterentwickelten Lipasen, insbesondere solche mit einem oder mehreren der folgenden Aminosäureaustausche ausgehend von der genannten Lipase in den Positionen D96L, T213R und/oder N233R, besonders bevorzugt T213R und N233R. Zu den bevorzugten kommerziellen Lipaseprodukten gehören Lipolase^{™}, Lipex^{™}, Lipolex^{™} und Lipoclean^{™} (Novozymes A/S), Lumafast (Genencor/DuPont) und Lipomax (Gist-Brocades).

Zur Erhöhung der bleichenden Wirkung können erfindungsgemäß Oxidoreduktasen, z.B. Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie beispielsweise bei der Konfektionierung von Wasch- und Reinigungsmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Ein weiterer Erfindungsgegenstand ist ein Verfahren zur Reinigung von Textilien und/oder harten Oberflächen, insbesondere Geschirr, das dadurch gekennzeichnet ist, dass in mindestens einem Verfahrensschritt ein erfindungsgemäßes Mittel angewendet wird. In verschiedenen Ausführungsformen zeichnet sich das beschriebene Verfahren dadurch aus, dass die Tannase bei einer Temperatur von etwa 0°C bis etwa 100°C, bevorzugt etwa 20°C bis etwa 60°C und weiter bevorzugt etwa 20°C bis etwa 40°C eingesetzt wird.

Hierunter fallen sowohl manuelle als auch maschinelle Verfahren, wobei maschinelle Verfahren aufgrund ihrer präziseren Steuerbarkeit, was z.B. die eingesetzten Mengen und Einwirkzeiten angeht, bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird.

Da erfindungsgemäße Tannasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass als einzige reinigungsaktive Komponente eine erfindungsgemäße Tannase mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Tannase aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Tannasen in Wasch- oder Reinigungsmitteln, beispielsweise wie oben beschrieben, zur (verbesserten) Entfernung von bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, beispielsweise von Textilien und/oder harten Oberflächen.

In einer weiteren bevorzugten Ausführungsform betrifft die Erfindung die Verwendung einer hierin beschriebenen erfindungsgemäßen Tannase in einem Wasch- oder Reinigungsmittel zur Verbesserung der Reinigungsleistung eines solchen tannasehaltigen Wasch- oder Reinigungsmittels, insbesondere eines flüssigen Wasch- oder Reinigungsmittels, an mindestens einer und zunehmend bevorzugt an zwei, drei, vier, fünf, sechs oder sieben tannasesensitiven Anschmutzung(en), die vorzugsweise aus der aus bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, wobei die Verbesserung der Reinigungsleistung eines Mittels mit erfindungsgemäßer Tannase gegenüber einem Mittel ohne Tannase, wie in Beispiel 2 beschrieben, bestimmt wird, insbesondere in einem Temperaturbereich von etwa 20°C bis etwa 40°C.

In einer weiteren bevorzugten Ausführungsform betrifft die Erfindung die Verwendung einer Tannase in einem Wasch- oder Reinigungsmittel, insbesondere flüssigen Wasch- oder Reinigungsmittel, zur Verbesserung der Reinigungsleistung eines solchen tannasehaltigen Wasch- oder Reinigungsmittels an mindestens einer und zunehmend bevorzugt an zwei, drei, vier, fünf, sechs oder sieben tannasesensitiven Anschmutzung(en), die vorzugsweise aus der aus bleichbaren und/oder tanninhaltigen Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind, wobei die Verbesserung der Reinigungsleistung eines Mittels mit Tannase gegenüber einem Mittel ohne Tannase, wie in Beispiel 2 beschrieben, bestimmt wird, insbesondere in einem Temperaturbereich von etwa 20°C bis etwa 40°C, wobei die Tannase eine Tannase ist, die tanninabbauende Aktivität aufweist und eine Aminosäuresequenz umfasst, 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% identisch ist.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren und Verwendungen gilt.

### BEISPIELE

**Tabelle1: Verwendete Waschmittelmatrix**

| Chemischer Name | Gew.-% Aktivsubstanz im Rohmaterial | Gew.-% Aktivsubstanz in der Formulierung |
|---|---|---|
| Wasser demin. | 100% | Rest |
| LAS | 96% | 3-20% |
| FAEOS | 70% | 3-8% |
| Palmkernölsäure | 30% | 0,3-4% |
| FAEO | 100% | 2-11 % |
| HEDP | 60% | 0,5 - <2% |
| Zitronensäure | 100% | 1-5% |
| NaOH | 50% | 0,5-2% |
| Entschäumer | 100% | <1% |
| Glycerin | 99,5% | 1-3% |
| 1,2-Propandiol | 100% | 8-12% |
| Monoethanolamin | 100% | 4-8% |
| Soil repellent polymer | 30% | 0,5-1% |
| Stabilisator | 100% | 0,3-1% |
| Perfumes, DTI | t.q. | minors |
| Dosierung 3,17 g/L; pH 8,2 bis 8,4 | | |

### Beispiel 1: Bestimmung der Tannaseaktivität

Die rekombinante Expression der Tannase gemäß SEQ ID NO: 1 erfolgte gemäß in der Literatur beschriebenen Verfahren (Vojcic et al. (2012) An efficient transformation method for Bacillus subtilis DB104, Appl Microbiol Biotechnol 94(2): 487-493).

2 µl der transformierten Kulturüberstände wurden auf 1 % (w/v) Tanninsäure enthaltende LB-Agar-Platten (10 g/L Trypton, 10 g/L NaCl, 5 g/L Hefeextrakt, 14 g/L Agar) aufgetragen. Die Kulturen wurden für 17 h bei 37°C inkubiert. Anschließend wurden die Agar-Platten auf Hofbildungen untersucht. Die Bildung eines Hofes auf der Agar-Platte zeigt die Aktivität der Tannase an, da diese die in den Agar-Platten enthaltene Tanninsäure abbaut und so zu einer Entfärbung der Agar-Platte führt.

### Beispiel 2: Bestimmung der Reinigungsleistung

### Miniwaschtest

Die Reinigungsleistung einer Tannase gemäß SEQ ID NO:1 wurde in einem Waschmittel gemäß Tabelle 1 an sieben Anschmutzungen bestimmt.
Bedingungen: 40°C, 16° dH Wasser, 1 h
Aktivproteingehalt der eingesetzten Tannase: 0,1 µg pro 1 ml Waschlauge
Anschmutzungen:
   1. CFT CS-03, Wine aged
   2. CFT CS-103, Wine unaged
   3. CFT C-BC-01, Tea, high/medium T
   4. WFK 10 JB, Black currant juice
   5. WFK 10 BB, Blackberry juice
   6. EMPA E167, Tea
   7. CFT C-BC-02, Coffee

Ausgestanzte Gewebe (Durchmesser = 10 mm) wurden in 48-Well Mikrotiterplatten vorgelegt, die Waschlauge wurde auf pH = 8 eingestellt und auf 40°C vortemperiert, Endkonzentration 3,17 g/L. Waschlauge ohne Enzyme und Waschlauge mit Tannase-Überstand aus Beispiel 1 wurden auf die Anschmutzung gegeben und für 60 min bei 40°C und 600 rpm inkubiert. Anschließend wurde die Anschmutzung mehrmals mit klarem Wasser gespült, getrocknet und mit einem Farbmessgerät (MACH 5) wurde die Helligkeit bestimmt. Je heller das Gewebe, desto besser die Reinigungsleistung. Gemessen wird hier der Y-Wert = Helligkeit, je höher desto heller. Um die Reinigungsleistung der erfindungsgemäßen Tannase mit der Waschlauge ohne Tannase zu vergleichen, wurde die Reinigungsleistung der Waschlauge ohne Tannase auf 100% normiert und die relative Reinigungsleistung der erfindungsgemäßen Tannase dazu im Vergleich berechnet.

Die Ergebnisse sind in den Tabelle 2 zusammengefasst.

**Tabelle 2: Waschleistung in % bei 40°C:**

| | Ohne Tannase | Mit Tannase |
|---|---|---|
| CFT CS-03 | 100% | 103% |
| CFT CS-103 | 100% | 107% |
| CFT CBC-01 | 100% | 103% |
| WFK 10 JB | 100% | 103% |
| WFK 10 BB | 100% | 109% |
| EMPA E167 | 100% | 102% |
| CFT CBC-02 | 100% | 103% |
| % Gesamtleistung | 100% | 104% |

Die erfindungsgemäße Tannase zeigt eine signifikant verbesserte Waschleistung an verschiedenen Anschmutzungen im Vergleich zur Waschlauge ohne Tannase.

## Patentansprüche

1. Tannase, umfassend eine Aminosäuresequenz, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität mit der in SEQ ID NO:1 angegebenen Aminosäuresequenz über deren Gesamtlänge aufweist, oder Varianten davon.

2. Tannase oder Varianten davon, **dadurch gekennzeichnet, dass**
(a) sie aus einer Tannase nach Anspruch 1 als Ausgangsmolekül erhältlich ist/sind durch ein- oder mehrfache konservative Aminosäuresubstitution(en); und/oder
(b) sie aus einer Tannase nach Anspruch 1 als Ausgangsmolekül erhältlich ist/sind durch Fragmentierung, Deletions-, Insertions- oder Substitutionsmutagenese und eine Aminosäuresequenz umfassen, die über eine Länge von mindestens 200, 210, 220, 230, 240, 250, 260, 270, 280, 290, 300,310, 320, 330,340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit dem Ausgangsmolekül übereinstimmt.

3. Tannase nach Anspruch 1 oder 2, wobei die Tannase an mindestens einer und zunehmend bevorzugt an zwei, drei, vier, fünf, sechs oder sieben tannasesensitiven Anschmutzung(en), die vorzugsweise aus der aus bleichbaren und/oder tanninhaltigen und/oder tanninderivathaltigen Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind, eine verbesserte Reinigungsleistung eines Wasch- oder Reinigungsmittels, welches eine erfindungsgemäße Tannase enthält, gegenüber einem Mittel ohne Tannase bewirkt, wobei die Reinigungsleistung, wie in Beispiel 2 beschrieben, bestimmt wird.

4. Verfahren zur Herstellung einer Tannase nach einem der vorhergehenden Ansprüche, umfassend das Bereitstellen einer Ausgangstannase, die mindestens 70% und zunehmend bevorzugt 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 98,6%, 98,7%, 98,8%, 98,9%, 99,0%, 99,1%, 99,2%, 99,3%, 99,4%, 99,5%, 99,6%, 99,7%, 99,8%, 99,9% oder 100% Sequenzidentität zu der in SEQ ID NO:1 angegebenen Aminosäuresequenz über deren Gesamtlänge aufweist.

5. Verfahren nach Anspruch 4, ferner umfassend einen oder mehreren der folgenden Verfahrensschritte:
(a) Einbringen einer ein- oder mehrfachen konservativen Aminosäuresubstitution(en) in die Ausgangstannase;
(b) Veränderung der Aminosäuresequenz der Ausgangstannase durch Fragmentierung, Deletions-, Insertions- oder Substitutionsmutagenese derart, dass die veränderte Tannase eine Aminosäuresequenz umfasst, die über eine Länge von mindestens 200, 210, 220, 230, 240, 250, 260, 270, 280, 290, 300,310, 320, 330,340, 350, 360, 370, 380, 390, 400, 410, 420, 430, 440, 450, 460, 470, 480, 490, 491, 492, 493, 494, 495, 496, 497, 498, 499, 500, 501, 502, 503, 504, 505, 506, 507, 508, 509, 510 oder 511 zusammenhängenden Aminosäuren mit der Ausgangstannase übereinstimmt.

6. Nukleinsäure codierend für eine Tannase nach einem der Ansprüche 1 bis 3 oder codierend für eine nach einem Verfahren der Ansprüche 4 bis 5 erhaltene Tannase.

7. Vektor enthaltend eine Nukleinsäure nach Anspruch 6, insbesondere ein Klonierungsvektor oder ein Expressionsvektor.

8. Nicht-menschliche Wirtszelle, die eine Nukleinsäure nach Anspruch 6 oder einen Vektor nach Anspruch 7 beinhaltet, oder die eine Tannase nach einem der Ansprüche 1 bis 3 beinhaltet, oder die eine nach einem Verfahren der Ansprüche 4 bis 5 erhaltene Tannase beinhaltet, insbesondere eine, die die Protease in das die Wirtszelle umgebende Medium sezerniert.

9. Verfahren zur Herstellung einer Tannase umfassend
a) Kultivieren einer Wirtszelle gemäß Anspruch 8 und
b) Isolieren der Protease aus dem Kulturmedium oder aus der Wirtszelle.

10. Mittel, insbesondere Wasch- oder Reinigungsmittel, insbesondere flüssiges Wasch- oder Reinigungsmittel, **dadurch gekennzeichnet, dass** es mindestens eine Tannase nach einem der Ansprüche 1 bis 3 oder eine nach einem Verfahren der Ansprüche 4 bis 5 erhaltene Protease enthält, wobei die Konzentration der mindestens einen Tannase insbesondere von 0,00005 bis 15 Gew.-%, vorzugsweise von 0,0001 bis 5 Gew.-%, weiter bevorzugt von 0,001 bis 1 Gew.-% und besonders bevorzugt von 0,001 bis 0,1 Gew.-%, bezogen auf aktives Protein, beträgt und/oder wobei das Mittel im Wesentlichen frei von borhaltigen Verbindungen ist und/oder wobei das Mittel einen pH-Wert von etwa 8 bis etwa 9 aufweist und/oder wobei das Mittel im Wesentlichen frei von phosphonathaltigen Verbindungen ist und/oder wobei das Mittel im Wesentlichen frei von phosphathaltigen Verbindungen ist.

11. Verfahren zur Reinigung von Textilien oder harten Oberflächen, **dadurch gekennzeichnet, dass** in mindestens einem Verfahrensschritt ein Mittel nach Anspruch 10 angewendet wird, oder dass in mindestens einem Verfahrensschritt eine Tannase nach einem der Ansprüche 1 bis 3 oder eine nach einem Verfahren nach Anspruch 4 oder 5 erhaltene Tannase katalytisch aktiv wird.

12. Verwendung einer Tannase nach einem der Ansprüche 1 bis 3 oder eine nach einem Verfahren der Ansprüche 4 bis 5 erhaltene Tannase in einem Wasch- oder Reinigungsmittel, insbesondere flüssigen Wasch- oder Reinigungsmittel, zur Entfernung von bleichbaren und/oder tanninhaltigen und/oder tanninderivathaltigen Anschmutzungen, die insbesondere von Früchten, Fruchtsäften, Nüssen, Hülsenfrüchten, Tee, Kaffee, Wein, Kakao oder Schokolade hervorgerufen werden.

13. Verwendung einer Tannase nach einem der Ansprüche 1 bis 3 oder eine nach einem Verfahren der Ansprüche 4 bis 5 erhaltene Tannase in einem Wasch- oder Reinigungsmittel, insbesondere flüssigen Wasch- oder Reinigungsmittel, zur Verbesserung der Reinigungsleistung eines solchen tannasehaltigen Wasch- oder Reinigungsmittel an mindesten einer und zunehmend bevorzugt an zwei, drei, vier, fünf, sechs oder sieben tannasesensitiven Anschmutzung(en), die vorzugsweise aus der aus bleichbaren und/oder tanninhaltigen und/oder tanninderivathaltigen Anschmutzungen, die insbesondere von Früchten, Fruchtsäften, Nüssen, Hülsenfrüchten, Tee, Kaffee, Wein, Kakao oder Schokolade hervorgerufen werden, bestehenden Gruppe ausgewählt ist/sind, wobei die Verbesserung der Reinigungsleistung eines Mittels mit erfindungsgemäßer Tannase gegenüber einem Mittel ohne Tannase, wie in Beispiel 2 beschrieben, bestimmt wird, insbesondere in einem Temperaturbereich von etwa 20°C bis etwa 40°C.
